# EUROPEAN PATENT APPLICATION

(11) **EP 4 484 560 A1**
(43) Date of publication of application: **01.01.2025**
(21) Application number: 23759950.1
(22) Date of filing: 21.02.2023
(51) Int. Cl.: C12N 15/01, A01H 1/00, A01H 1/06, A01H 5/00, A01H 5/12, A01H 6/82, A24B 15/10, C12N 15/09, C12N 15/29

(54) **NICOTIANA PLANT BODY AND METHOD FOR PRODUCING SAME**

(30) Priority: 22.02.2022 JP 2022026111
(71) Applicant: Japan Tobacco Inc., Tokyo 105-6927 (JP)
(72) Inventor: MAGOME, Hiroshi, Tokyo 130-8603 (JP); OYAMA, Kiyoshi, Tokyo 130-8603 (JP); TAKAKURA, Yoshimitsu, Tokyo 130-8603 (JP)
(74) Representative: Hoffmann Eitle
(86) International application number: PCT/JP2023/006116
(87) International publication number: WO 2023/162949

(57) **Abstract**

In a tobacco plant according to the present embodiment, a mutation that causes suppression of a function of a first endogenous gene which contains a first polynucleotide as a coding region and a function of a second endogenous gene which contains a second polynucleotide as a coding region is introduced in a genome.

## Description

### Technical Field

The present invention relates to (i) a tobacco plant which improves quality of tobacco leaves, (ii) a method for producing the tobacco plant, and (iii) a harvest from the tobacco plant and a processed product of the harvest.

### Background Art

The term "carotenoid" is a collective term for biopigments that exhibit red, yellow, or orange. Examples of a plant carotenoid include β-carotene and lutein. Degraded products of carotenoids are collectively referred to as "apocarotenoids". Examples of the apocarotenoids, which refer to the degraded products of carotenoids, include aroma constituents (for example, β-damascenone, β-ionone, and megastigmatrienone).

It is known that with respect to tobacco smoke obtained from a tobacco material containing apocarotenoids, various aromas such as fruity, floral, woody, and tobacco aromas are conferred. Research and development for increasing a content of apocarotenoid in a tobacco plant as well as producing a tobacco material by adding an apocarotenoid(s) as an aroma chemical are underway.

For example, Patent Literature 1 discloses, as a method for adjusting the content of β-damascenone in a tobacco plant, the method including the step of adjusting, by a genetic modification technology, expression or activity of neoxanthin synthase in the plant.

Patent Literature 2 discloses improving a content of carotenoid in a tobacco leaf and qualities of taste and aroma presented by a tobacco leaf, by applying a carotenoid synthesis activator to a surface of a tobacco plant or tobacco leaf so that the carotenoid synthesis activator is involved in processes of absorption, degradation, transformation, and synthesis of the tobacco plant.

Non-Patent Literature 1 discloses changing the content of carotenoid, by inhibiting expression of an LCY-e gene of a tobacco plant by virus-induced silencing.

### Citation List

### [Patent Literature]

[Patent Literature 1]
   Published Japanese Translation of PCT International Application Tokuhyo No. 2014-539311
[Patent Literature 2]
   Chinese Patent Application Publication No. 101322463

### [Non-patent Literature]

[Non-patent Literature 1]
Molecular Cloning and Functional Characterization of the Lycopene ε-Cyclase Gene via Virus-Induced Gene Silencing and Its Expression Pattern in *Nicotiana tabacum* (2014)

### Summary of Invention

### Technical Problem

Administration of a chemical agent to a tobacco plant or a tobacco leaf has a problem in elimination of the chemical agent from a plant body and a product. Further, in a case where an exogenous gene is introduced into a plant by a genetic modification technology, there is a problem in that a position where the exogenous gene is to be inserted cannot be controlled.

Furthermore, sufficiently increasing the content of apocarotenoid in a tobacco plant by controlling gene expression has not yet been achieved. Therefore, further research and development are demanded.

An object of an aspect of the present invention is to provide a tobacco plant that makes it possible to improve quality of a tobacco product.

### Solution to Problem

In order to solve the above problems, the present inventors made diligent studies. As a result, the inventors have found that quantities of apocarotenoids increase in a tobacco plant whose specific endogenous genes are subjected to suppression of a function. The inventors have thus accomplished the present invention.

In other words, a mutation that causes suppression of a function of a first endogenous gene and a function of a second endogenous gene is introduced in a genome, the first endogenous gene containing, as a coding region, a first polynucleotide that encodes a first polypeptide having a sequence identity of 95% or higher with an amino acid sequence represented by SEQ ID NO: 1; and the second endogenous gene containing, as a coding region, a second polynucleotide that encodes a second polypeptide having a sequence identity of 95% or higher with an amino acid sequence represented by SEQ ID NO: 2.

Further, included is a step of introducing, in a genome of a tobacco plant, a mutation that causes suppression of a function of a first endogenous gene and a function of a second endogenous gene, the first endogenous gene containing, as a coding region, a first polynucleotide that encodes a first polypeptide having a sequence identity of 95% or higher with an amino acid sequence represented by SEQ ID NO: 1; and the second endogenous gene containing, as a coding region, a second polynucleotide that encodes a second polypeptide having a sequence identity of 95% or higher with an amino acid sequence represented by SEQ ID NO: 2.

### Advantageous Effects of Invention

An aspect of the present invention makes it possible to improve quality of a tobacco leaf harvested from a tobacco plant.

### Brief Description of Drawings

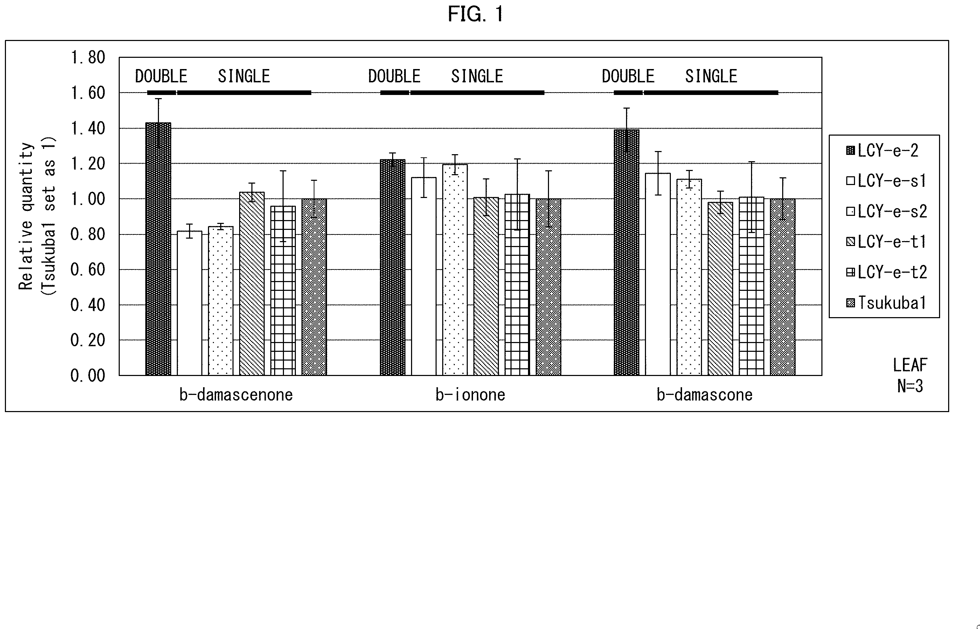

Fig. 1 is a chart showing analysis results of apocarotenoids in a double mutant, a single mutant, and Tsukuba 1.

### Description of Embodiments

### [1. Tobacco plant]

In a tobacco plant in accordance with an aspect of the present invention (hereinafter, which may also be referred to as "the present tobacco plant"), a mutation that causes suppression of a function of a first endogenous gene and a function of a second endogenous gene is introduced in a genome.

The term "tobacco plant" as used herein encompasses (i) individuals as a whole (such as a mature plant, a seedling, and a seed), (ii) tissues (such as a leaf, a stem, a flower, a root, a reproductive organ, an embryo, and a part of any of these), and (iii) cured products of any of these.

The term "endogenous gene" as used herein means a gene which is originally present in a genome of a tobacco plant. That is, an endogenous gene is not an exogenous gene which is present in a plant other than a tobacco plant.

The first endogenous gene contains, as a coding region, a first polynucleotide that encodes a first polypeptide having a sequence identity of 95% or higher with an amino acid sequence represented by SEQ ID NO: 1. The second endogenous gene contains, as a coding region, a second polynucleotide that encodes a second polypeptide having a sequence identity of 95% or higher with an amino acid sequence represented by SEQ ID NO: 2. The first endogenous gene is different from the second endogenous gene.

Further, the first endogenous gene includes, as a coding region, a first polynucleotide having a sequence identity of 93% or higher with a nucleotide sequence represented by SEQ ID NO: 3. The second endogenous gene includes, as a coding region, a second polynucleotide having a sequence identity of 93% or higher with a nucleotide sequence represented by SEQ ID NO: 4.

The first polynucleotide also encompasses a degenerate sequence of the nucleotide sequence represented by SEQ ID NO: 3, due to a degeneracy of the genetic code. The second polynucleotide also encompasses a degenerate sequence of the nucleotide sequence represented by SEQ ID NO: 4, due to a degeneracy of the genetic code.

A region that is present in the endogenous gene and that encodes a polypeptide is herein described as a coding region (CDS). Further, the terms "polypeptide" and "protein" herein have substantially the same meaning, and can therefore be used interchangeably.

The term "sequence identity" herein means a percentage at which a concerned sequence matches a reference sequence (a nucleotide sequence, an amino acid sequence, and the like). Note, here, that a part of the sequence which part does not match is a part at which a substitution, addition, deletion, or insertion is present.

The wording "polynucleotide having a sequence identity of 93% or higher with a nucleotide sequence represented by [...]", which specifies a polynucleotide with use of one of nucleotide sequences listed in a sequence listing, herein means a wild-type polynucleotide. The wild-type polynucleotide means a polynucleotide which is typically present in a tobacco plant described later.

SEQ ID NO: 1 represents an amino acid sequence of an LCY-e-S protein. SEQ ID NO: 2 represents an amino acid sequence of an LCY-e-T protein. SEQ ID NO: 3 represents a nucleotide sequence of a CDS (coding region) of an LCY-e-S gene. SEQ ID NO: 4 represents a nucleotide sequence of a CDS of an LCY-e-T gene.

The LCY-e-S gene and the LCY-e-T gene are each a gene that encodes a lycopene epsilon-cyclization enzyme (LCY-e). LCY-e is an enzyme that catalyzes a reaction in which an all-trans lycopene is used as a substrate and an epsilon ring is formed at a terminus thereof.

As described below, *Nicotiana tabacum* is an amphidiploid and has a genome derived from its parent plant *Nicotiana sylvestris* (also referred to as an "S genome") and a genome derived from its parent plant *Nicotiana tomentosiformis* (also referred to as a "T genome"). In a case where the tobacco plant is *Nicotiana tabacum,* an apocarotenoid content is increased by specifically suppressing functions of LCY-e genes of the S genome and the T genome (LCY-e-S gene and LCY-e-T gene).

In the present tobacco plant, each gene corresponding to the endogenous gene whose function is suppressed by the mutation mentioned above is highly conserved and has a high sequence identity. Nucleotide sequences of the LCY-e-S gene and the LCY-e-T gene have a homology of 96% and amino acid sequences of the LCY-e-S gene and the LCY-e-T gene have a homology of 98%.

The first polynucleotide only needs to be a polynucleotide that encodes a polypeptide having a sequence identity of 95% or higher with an amino acid sequence of an LCY-e-S protein, and the second polynucleotide only needs to be a polynucleotide that encodes a polypeptide having a sequence identity of 95% or higher with an amino acid sequence of an LCY-e-T protein. The sequence identity is preferably higher (96%, 97%, 98%, or 99% or higher).

The content of apocarotenoid in the present tobacco plant is increased as compared with a wild-type tobacco plant. In a specific embodiment, the "wild-type tobacco plant" is a tobacco plant in which the LCY-e-S gene and the LCY-e-T gene that are present in a genome of the wild-type tobacco plant are normally functioning. The wording "the LCY-e-S gene (and the LCY-e-T gene) is/are normally functioning" means that a factor which suppresses expression of the LCY-e-S gene (and the LCY-e-T gene) has not been introduced into the genome and that the LCY-e-S gene (and the LCY-e-T gene) is/are not mutated. Hereinafter, the "wild-type tobacco plant" may be referred to as "wild-type plant".

Examples of apocarotenoid encompass β-damascenone, P-ionone, β-damascone, dihydroactinidiolide, 3-hydroxy-β-damascone, 3-hydroxy-5,6-epoxy-β-ionol, β-cyclocitral, 3-hydroxy-7,8-dehydro-β-ionol, blumenol C, and the like. The present tobacco plant preferably has an increased content of at least one apocarotenoid selected from the group consisting of β-damascenone, β-ionone, and β-damascone as compared with a wild-type tobacco plant. The present tobacco plant more preferably has increased contents of β-damascenone and β-damascone as compared with a wild-type tobacco plant. More preferably, the present tobacco plant has an increased content of β-damascenone as compared with a wild-type tobacco plant.

The content of apocarotenoid in the present tobacco plant has been increased by 10% or more, 20% or more, or 30% or more, as compared with the content of apocarotenoid in the wild-type tobacco plant. Such a tobacco plant may be a cured product.

The content of apocarotenoid contained in the tobacco plant can be measured by, for example, gas chromatography-mass spectrometry (GC-MS).

In the present tobacco plant, polypeptides whose amounts are decreased as compared with that in a wild-type tobacco plant only need to be polypeptides each having a sequence identity of 95% or higher with an amino acid sequence of an LCY-e-S protein or an LCY-e-T protein. Further, the sequence identity is preferably higher (96%, 97%, 98%, or 99% or higher).

The wording "suppression of a function of an (endogenous) gene" as used herein means a state in which a gene that is originally present in a genome does not fulfill its original function. Therefore, the wording "suppression of a function of an (endogenous) gene" encompasses (i) "disruption of an (endogenous) gene", (ii) "a mutation of an (endogenous) gene", and (iii) "suppression of expression of an (endogenous) gene" by a gene (including an exogenous gene) other than the (endogenous) gene.

The "disruption of an (endogenous) gene" means that (i) a gene which is originally present in a genome is not present in the genome or (ii) a transcription product is not produced from a gene which is present in a genome. The "mutation of an (endogenous) gene" means, for example, (i) a mutation of a gene (i.e., decrease or impairment of a function) such that an original functional polypeptide is not produced, (ii) a mutation of a gene such that although a functional polypeptide is produced, the amount of the functional polypeptide produced is decreased, or (iii) a mutation of a gene such that although a functional polypeptide is produced, the stability of the functional polypeptide is decreased. The "suppression of expression of an (endogenous) gene" means, for example, a state in which although no change has occurred to a base of the (endogenous) gene, the transcriptional or translational function of the gene (from transcription into mRNA to subsequent translation into a polypeptide) is modified through another factor so that (i) the amount of the polypeptide produced is decreased or (ii) no polypeptide is produced. The "suppression of expression of an (endogenous) gene" can occur as a result of, for example, degradation of mRNA which is transcribed from the (endogenous) gene.

As used herein, the "mutation" has the meaning ordinarily understood in the technical field to which the present application belongs, and means, for example, any change in a base present in a wild-type genome or any change in an amino acid residue present in a wild-type polypeptide (examples of these changes encompass substitution, deletion, insertion, addition, duplication, inversion, and translocation). Therefore, the "mutation of an (endogenous) gene" means, for example, (i) a mutation of a gene such that an original functional polypeptide is not produced (including a mutation such that a polypeptide the function of which is decreased or impaired is produced), (ii) a mutation of a gene such that although a polypeptide is produced, the amount of the polypeptide produced is decreased, (iii) a mutation of a gene such that although a polypeptide is produced, the stability of the polypeptide is decreased, or (iv) a mutation of a gene such that the gene (a coding region or a genomic DNA sequence including an untranslated region) is lost or that transcription from the gene is suppressed (e.g., a transcription-regulating region or a transcription-initiating region is deleted).

In a case where a function is impaired by substitution, the substitution can be present in at least one of the following: a promoter sequence (a sequence upstream (5' side) of a coding region) and a sequence downstream (3' side) of the coding region); a 5' untranslated region and a 3' untranslated region; conserved sequences (such as GT at the 5' end and AG at the 3' end) present at both ends of an intron; and the coding region.

For example, substitution in a nucleotide sequence which is present in a promoter sequence, a 5' untranslated region, or a 3' untranslated region of a gene and which is important in regulating expression of the gene leads to a decrease in the transcriptional activity of the gene or to a decrease in the stability of a transcription product produced from the gene. Any of these decreases can lead to a reduction in transcription product produced from the gene, and ultimately lead to a reduction in translation product. Substitution in a conserved sequence present in an intron (splicing mutation) leads to splicing abnormality of mRNA. This results in abnormal mRNA in which an unnecessary intron is added or inserted. The abnormal mRNA generates an abnormal translation product or translation thereof does not terminate, due to, for example, frame shifting.

In a case where a nucleotide substitution present in a coding region is a missense mutation, the substitution leads to production of an amino acid different from an original amino acid. This results in a polypeptide the original function of which is decreased or impaired.

Substitution in a coding region can lead to production of a translation product which has an incomplete length or a translation product which does not maintain an original function. The translation product which has an incomplete length is derived from conversion, by the substitution, of a codon which encodes an amino acid into a stop codon (i.e., nonsense mutation). As compared with an original translation product, the translation product which has an incomplete length is such that one or more consecutive amino acid residues including an amino acid residue at a C-terminus are deleted. The nonsense mutation occurs to any codon present upstream of an original stop codon, and is preferably present upstream of the original stop codon with one or more codons therebetween. Thus, a translation product produced from a gene which has a nonsense mutation has an incomplete length. The translation product which does not maintain an original function is produced due to amino acid substitution. In this case, the amount of a transcription product may be equal to that of a transcription product in a wild-type plant. The translation product has, therein, a change in tertiary structure, a decrease in function as a functional domain, or the like. A preferred aspect of the mutation of the present invention is such amino acid substitution that leads to production of a translation product which does not maintain an original function. The amino acid substitution is preferably non-conservative substitution with a high possibility of changing the function of the translation product. Examples of the non-conservative substitution encompass (i) substitution of an amino acid by another amino acid having a different electric charge or different hydrophobicity (e.g., substitution of a basic amino acid by an acidic amino acid, substitution of a basic amino acid or an acidic amino acid by a neutral amino acid, substitution of a neutral amino acid by a basic amino acid or an acidic amino acid, and substitution of a polar amino acid by a non-polar amino acid) and (ii) substitution of an amino acid by another amino acid having a side chain of a different bulk (three-dimensional size).

As another example of a phenomenon caused by a nonsense mutation, in a case where a protein coding region of an LCY-e-S gene or an LCY-e-T gene has a nonsense mutation, nonsense-mediated mRNA decay (Brogna and Wen (2009) Nat. Structural Mol. Biol. 16: 107-113) can occur. The nonsense-mediated mRNA decay leads to degradation of a transcription product. Thus, the nonsense mutation may lead to a decrease in the amount of the transcription product. In a case where a target gene is composed of a plurality of exons, it is preferable that at least one exon which has the nonsense mutation be present, and it is more preferable that the exon which has the nonsense mutation be not, among the plurality of exons of which the target gene is composed, an exon which is present most downstream (3' side), in order to cause the nonsense-mediated mRNA decay.

In a case where a mutation(s) (deletion, insertion, and/or the like) other than substitution occur(s) in a promoter sequence, a 5' untranslated region, and/or a 3' untranslated region, a decrease can occur in transcriptional activity or stability as in the case of the substitution, so that (i) the amount of a transcription product can decrease and (ii) the amount of a polypeptide can decrease. In addition, a mutation, other than substitution, in a conserved sequence present in an intron can lead to translation into a polypeptide having an amino acid sequence different from an original amino acid sequence, as in the case of the substitution. A mutation, other than substitution, in a coding region can lead to translation into a polypeptide having an amino acid sequence different from an original amino acid sequence, due to (i) deletion or insertion of an amino acid residue (caused by deletion or insertion of consecutive bases which are multiples of 3) or (ii) frame shifting. Large deletion of an entire gene or insertion of a large fragment into the gene can cause expression itself of the gene to be lost.

An individual, which is generated as a result of a mutation or disruption of the endogenous genes described above, is herein referred to as a mutant of a tobacco plant (hereinafter simply referred to as "mutant"). Note that a single gene may have a single mutation or a plurality of mutations for causing impairment of a function and the mutation(s) can be any mutation(s).

The mutant is preferably in any one of the states (i) to (iv). In a mutant that is in any one of the states (i) to (iv), the function is substantially lost. Then, the object of the mutation or the disruption (production of a tobacco plant having an increased content of apocarotenoid as compared with a wild-type plant) can be sufficiently and reliably achieved. From a viewpoint that a tobacco plant having a high apocarotenoid content can be obtained, the mutant is preferably a mutant in the state (ii), (iii), or (iv).
(i) The first endogenous gene and/or the second endogenous gene is/are a mutated gene(s) in which each of alleles has the above mutation.
(ii) Both the first endogenous gene and the second endogenous gene are mutated genes in each of which both alleles have the above mutation.
(iii) Both the first endogenous gene and the second endogenous gene are mutated genes in each of which alleles have the same type of mutation or have the above mutation in the same position.
(iv) Both the first endogenous gene and the second endogenous gene are homozygotes in each of which alleles have the same type of mutation.

A homozygote of a mutated gene herein only needs to be a zygote of mutated genes in which the position and type of the mutation are the same.

Suppression of expression of each of the endogenous genes encompass (i) suppression of transcription from the endogenous gene to mRNA, (ii) suppression of translation from the endogenous gene into a polypeptide through mRNA (e.g., degradation of the mRNA), and (iii) suppression of the function of the polypeptide which has been produced by the translation. The degradation of the mRNA can occur due to the nonsense-mediated mRNA decay. The suppression of the transcription can be achieved by, for example, (i) inhibition of a transcription factor which promotes the transcription from the endogenous gene or (ii) inhibition of access of a transcription initiation factor to the endogenous gene. The suppression of the translation can be achieved with use of an antisense RNA molecule, an RNAi molecule, or a co-suppression molecule. The suppression of the function of the polypeptide can be achieved by a molecule which binds to a polypeptide that is functional and thereby inhibits the function of the polypeptide. Examples of such a molecule encompass decoy nucleic acids, ribozymes, antibodies, and inhibitory peptides.

The above suppression (of the transcription, the translation, or the function of the polypeptide) can be achieved by, for example, (i) directly introducing a molecule for achieving the suppression into a plant or (ii) introducing, into a plant, a nucleic acid molecule encoding the molecule (i.e., transformation of the plant). Note, here, that as a result of the transformation of the plant, the nucleic acid molecule is incorporated into any one or more regions of a genome of the plant. Provided that the suppression is achieved, it is unnecessary for the nucleic acid molecule to be incorporated into both the S genome and the T genome as a result of the transformation of the plant.

In the tobacco plant, the suppression of the function is preferably a decrease in the amount of the polypeptide, which is a product of expression of the endogenous gene, as compared with a wild-type plant. Specifically, the amount is decreased through mutation which leads to suppression of a function of an endogenous gene encoding the wild-type polypeptide.

The "decreased amount" of the polypeptide means that the polypeptide is present at a percentage of 70% or less, 60% or less, 50% or less, 40% or less, 30% or less, 20% or less, 10% or less, 5% or less, or 1% or less with respect to the amount of a wild-type polypeptide. The amount of the polypeptide with respect to the amount of the wild-type polypeptide can be selected, as appropriate, from the above-described values which cause a content of at least one apocarotenoid in the tobacco plant to change.

Note that it is preferable that the above-described decrease in the amount of the polypeptide present in the tobacco plant be genetically and stably inherited by a cultured cell, a callus, protoplast, a seed, and offspring each of which is obtained from the tobacco plant. Therefore, the tobacco plant can be an individual developed from the cultured cell, the callus, the protoplast, the seed, or the offspring, each of which has been produced through an artificial operation. Thus, these materials, from each of which the individual develops, are also encompassed in the scope of the present invention.

The tobacco plant can further encompass bred progeny obtained by crossing. Breeding with use of mutants has been performed in many plant species, including rice, wheat, barley, and soybean. For example, a mutant isolated from a mutant population which has been obtained by a treatment with use of a mutagen has multiple mutations in a region other than a region of a target gene. In general, therefore, backcrossing is performed to remove an excess mutation(s). In this crossing, a desired character (increased content of apocarotenoid) of the mutant can be introduced into an existing cultivar by crossing the mutant with the cultivar having an excellent character. Bred progeny thus obtained can be a variety obtained by adding high values to an existing cultivar.

Note that the desired character of the mutant is derived from mutations introduced into a plurality of positions (e.g., a plurality of genes) on a genome. For efficient backcrossing, it is therefore necessary to select, in advance, individuals having the mutations. In the selection of the individuals, it is advantageous to be able to easily detect (i) whether or not the mutations are present in the individuals and (ii) whether the mutations are homozygous or heterozygous. The mutations can be detected by a method (described later) for detecting mutations in genes. Apart from the perspective above, it is preferable that lines having a high cultivar-return-rate (i.e., a high proportion of a cultivar-derived genomic region to the entire genomic region) be obtained with the fewer times of crossing. Fewer times of crossing can be achieved by, for example, Marker Assisted Selection (MAS) which uses a background marker indicative of a polymorphism between the mutant and the existing cultivar. The background marker indicative of a polymorphism can be, for example, SNP or Simple Sequence Repeat (SSR) each of which is known in tobacco. Other than the existing marker, examples of a new marker encompass the following differences (a) and (b) which are identified by determining respective genome sequences of the mutant and the existing cultivar for use in crossing and then making a comparison between the genome sequences: (a) a difference in nucleotide sequence and (b) a difference in the number of repeat sequences on a genome.

Respective polypeptides encoded by the first polynucleotide and the second polynucleotide are each a polypeptide (or a variant thereof) that is present in a wild-type plant. Therefore, the amount of the polypeptide in the tobacco plant is decreased in comparison with that of a wild-type plant. This causes the tobacco plant to be inferior to the wild-type plant in terms of the function. The function is, for example, a function for catalyzing a reaction in which an all-trans lycopene is used as a substrate and an epsilon ring is formed at a terminus thereof.

In the tobacco plant, the suppression of the function is preferably a decrease in the amount of translation into the polypeptide, which is a product of expression of the endogenous gene, as compared with a wild-type plant. The translation into the polypeptide occurs based on (i) a decrease in mRNA (due to, for example, the amount of the mRNA which amount results from the instability of the mRNA itself, promotion of degradation of the mRNA, or suppression of the transcription of the mRNA) or (ii) a decrease in the amount of the translation of the mRNA (due to, for example, lack of elements (tRNA and ribosome) involved in the translation, inhibition of recruit, or functional impairment).

In the tobacco plant, the suppression of the function is preferably a decrease in the amount of the mRNA which has been transcribed from the endogenous gene, as compared with a wild-type plant. The decrease in the amount of the mRNA occurs due to, for example, suppression of the transcription of the mRNA from the endogenous gene. The suppression of the transcription can be achieved by, for example, inhibition of access of a transcription initiation factor to the endogenous gene, which occurs as a result of introduction of the mutation into the endogenous gene.

In the tobacco plant, the suppression of the function is preferably promotion of degradation of the mRNA which has been transcribed from the endogenous gene. The degradation of the mRNA may be caused by, for example, (i) the presence, in the endogenous gene, of a nonsense mutation which causes nonsense-mediated mRNA decay, (ii) the presence of an exogenous factor leading to the degradation of the mRNA, (iii) activation of an endogenous constituent element leading to the degradation of the mRNA, or (iv) the presence of a sequence for promoting the degradation of the mRNA. In a case where the degradation of the mRNA which has been transcribed from the endogenous gene is promoted in the tobacco plant, the mRNA in the tobacco plant is decreased. That is, in the tobacco plant, the suppression of the function may be a decrease in the amount of the mRNA which has been transcribed from the endogenous gene, as compared with a wild-type plant. Note, here, that the wording "decrease in the amount of the mRNA which has been transcribed from the endogenous gene" means that such a transcription product is present at a percentage of 70% or less, 60% or less, 50% or less, 40% or less, 30% or less, 20% or less, 10% or less, 5% or less, or 1% or less with respect to the amount of a transcription product produced from the endogenous gene in a wild-type plant.

In the tobacco plant, the mutation may be insertion, into a region outside a region in which the endogenous gene is present, of a polynucleotide which expresses a factor that promotes the degradation of the mRNA that has been transcribed from the endogenous gene.

The factor is preferably an antisense RNA molecule, an RNAi molecule, or a co-suppression molecule.

In the tobacco plant, the mutation or the disruption of the endogenous gene occurs as a result of, for example, a spontaneous mutation, a mutagen treatment, genetic modification, genome editing, or gene knockout. The spontaneous mutation of the endogenous gene generally occurs due to (i) a replication error and (ii) damage to the endogenous gene. The cause of the damage is, for example, exposure to a publicly-known naturally-occurring mutagen (e.g., radiation or ultraviolet rays). The mutagen treatment with respect to the endogenous gene can be carried out by artificially causing the mutagen to act on the tobacco plant (as necessary, in combination with suppression of a gene repair function). Examples of the type of the mutagen encompass chemical agents such as ethyl methane sulfonate (EMS), sodium azide, ethidium bromide, and nitrous acid. Note, however, that the mutagen is not limited to these chemical agents, provided that the mutagen is a chemical agent which causes the mutation in a genomic DNA of a tobacco plant. Examples of the mutagen also encompass γ rays, heavy ion beams, X-rays, neutron beams, and UV rays. Note, however, that the mutagen is not limited to these beams and rays, provided that the mutagen is a radiation or the like which causes the mutation in a genomic DNA of a tobacco plant. The mutagen is preferably EMS. These methods are preferable from the viewpoint that an exogenous factor need not be added to a target plant. Modification of the endogenous gene can be carried out by homologously modifying part or the whole of a target gene with use of a modifying sequence according to a publicly-known genetic modification method. The genome editing of the endogenous gene can be carried out by a publicly-known technique (for example, zinc-finger nucleases (ZFN), transcription activator-like effector nucleases (TALEN), and a CRISPR/Cas9 system). The gene knockout can be carried out by, for example, insertion of a publicly-known transposon (mobile genetic factor) or T-DNA.

The various mutations described above can be easily introduced into a tobacco plant by a person skilled in the art who has referred to, for example, genome sequences of genes represented by SEQ ID NOs: 5 and 6. Specifically, on the basis of these pieces of sequence information, it is possible to appropriately determine a region which is present in a genome of any of various tobacco plants encompassed in the scope of the present invention and in which a mutation should be introduced. SEQ ID NO: 5 represents a genome nucleotide sequence of an LCY-e-S gene that is present in a genome of *Nicotiana tabacum* (Tsukuba 1). SEQ ID NO: 6 represents a genome nucleotide sequence of an LCY-e-T gene present in a genome of *Nicotiana tabacum* (Tsukuba 1). SEQ ID NOs: 5 and 6 include a 5' untranslated region and a 3' untranslated region (approximately 1 kb each).

There are methods well-known to a person skilled in the art for isolating homologous genes. Examples of such methods encompass a hybridization technique (Southern, E. M., Journal of Molecular Biology, Vol. 98, 503, 1975) and a polymerase chain reaction (PCR) technique (Saiki, R. K., et al. Science, vol. 230, 1350-1354, 1985, Saiki, R. K. et al. Science, vol. 239, 487-491, 1988). Therefore, a person skilled in the art can easily isolate a homologous gene of the LCY-e-S gene from various plants while, for example, (i) a polynucleotide having a nucleotide sequence shown in SEQ ID NO: 3 or a part of the polynucleotide is serving as a probe or (ii) oligonucleotide hybridizing with the polynucleotide under stringent conditions is serving as a primer. A person skilled in the art who read these descriptions can easily (i) isolate a homologous gene of the LCY-e-T gene on the basis of the nucleotide sequence of SEQ ID NO: 4 (or on the basis of a part of the nucleotide sequence).

The stringent conditions indicate, in general, conditions under which (i) a double-stranded polynucleotide which is specific to a nucleotide sequence is formed but (ii) formation of a double-stranded polynucleotide which is not specific to the nucleotide sequence is markedly suppressed. In other words, the stringent conditions can be such that hybridization is carried out at a temperature falling within a range from (i) a melting temperature (Tm) of a hybrid of nucleic acids which are highly homologous to each other (e.g., a double-stranded polynucleotide which perfectly matches a probe) to (ii) a temperature 15°C lower, preferably 10°C lower, more preferably 5°C lower than the melting temperature (Tm). For example, the stringent conditions can be such that hybridization is carried out, in a common buffer solution for hybridization, at 68°C for 20 hours. In one example, hybridization is carried out, in a buffer solution (0.25 M Na2HPO4; pH 7.2; 7% SDS; 1 mM EDTA; and 1× Denhardt's solution), at 60°C to 68°C, preferably 65°C, more preferably 68°C for 16 hours to 24 hours, and then washing is carried out, in a buffer solution (20 mM Na2HPO4; pH 7.2; 1% SDS; and 1 mM EDTA), at 60°C to 68°C, preferably at 65°C, more preferably at 68°C for 15 minutes. This washing is carried out twice. In another example, prehybridization is carried out overnight at 42°C in a hybridization solution (containing 25% formamide or 50% formamide (for a more stringent condition); 4× SSC (sodium chloride/sodium citrate); 50 mM Hepes pH 7.0; 10× Denhardt's solution; and 20 µg/ml denatured salmon sperm DNA), and then hybridization is carried out by adding a labeled probe thereto and keeping a resulting solution overnight at 42°C. In washing following the hybridization, conditions of a washing solution and a temperature are approximately "1× SSC, 0.1% SDS, 37°C", approximately "0.5× SSC, 0.1% SDS, 42°C" for a more stringent condition, approximately "0.2× SSC, 0.1% SDS, 65°C" for a still more stringent condition. It can be thus expected that as the conditions of the washing following the hybridization become more stringent, DNA having higher homology to a sequence of a probe is isolated. Note, however, that the above combinations of the conditions of the SSC, the SDS, and the temperature are merely examples. A person skilled in the art can achieve stringency similar to the above by appropriately combining the above or other elements (e.g., a probe concentration, a probe length, and a time period for a hybridization reaction) that determine the stringency of hybridization. For example, a person skilled in the art can easily obtain such a gene by referring to Molecular Cloning (Sambrook, J. et al., Molecular Cloning: a Laboratory Manual 2nd ed., Cold Spring Harbor Laboratory Press, 10 Skyline Drive Plainview, NY (1989)).

The tobacco plant is not particularly limited, provided that the tobacco plant is a plant belonging to the genus *Nicotiana.* Examples of the tobacco plant encompass *Nicotiana acaulis, Nicotiana acuminata, Nicotiana acuminata var. multzjlora*, *Nicotiana africana, Nicotiana alata, Nicotiana amplexicaulis, Nicotiana arentsii, Nicotiana attenuata, Nicotiana benavidesii, Nicotiana bigelovii, Nicotiana bonariensis, Nicotiana cavicola, Nicotiana clevelandii, Nicotiana cordifolia, Nicotiana corymbosa, Nicotiana debneyi, Nicotiana excelsior, Nicotiana forgetiana, Nicotiana fragrans, Nicotiana glauca, Nicotiana glutinosa, Nicotiana goodspeedii, Nicotiana gossei, Nicotiana ingulba, Nicotiana kawakamii, Nicotiana knightiana, Nicotiana langsdorfi, Nicotiana linearis, Nicotiana longiflora, Nicotiana maritima, Nicotiana megalosiphon, Nicotiana miersii, Nicotiana noctiflora, Nicotiana nudicaulis, Nicotiana obtusifolia, Nicotiana occidentalis, Nicotiana occidentalis subsp. Hesperis, Nicotiana otophora, Nicotiana paniculata, Nicotiana pauczjlora, Nicotiana petunioides, Nicotiana plumbaginifolia, Nicotiana quadrivalvis, Nicotiana raimondii, Nicotiana repanda, Nicotiana rosulata, Nicotiana rosulata subsp. Ingulba, Nicotiana rotundifolia, Nicotiana rustica, Nicotiana setchellii, Nicotiana simulans, Nicotiana solanifolia, Nicotiana spegauinii, Nicotiana stocktonii, Nicotiana suaveolens, Nicotiana sylvestris, Nicotiana tabacum, Nicotiana thyrsiflora, Nicotiana tomentosa, Nicotiana tomentosiformis, Nicotiana trigonophylla, Nicotiana umbratica, Nicotiana undulata, Nicotiana velutina, Nicotiana wigandioides,* and hybrids of tobacco plants. Among these tobacco plants, *Nicotiana tabacum* and *Nicotiana rustica,* each of which is used as a material to produce a tobacco leaf, are preferable.

### [2. Method of producing tobacco plant]

A method for producing a tobacco plant in accordance with an aspect of the present invention (hereinafter, which may also be referred to as "the present method for producing a tobacco plant") includes the step of introducing, in a genome, a mutation that causes suppression of a function of a first endogenous gene and a function of a second endogenous gene.

The step of introducing a mutation in a genome of a tobacco plant results in an increase in the content of apocarotenoid in the tobacco plant through suppression of the function of the first endogenous gene and the function of the second endogenous gene. An overview of changing the content of apocarotenoid is as described above. Therefore, as concrete examples of carrying out the step, the following description will discuss introduction of a mutation into the above-described endogenous genes, which is performed by use of a genome editing technique. Examples of a usable genome editing technique encompass CRISPR/Cas9 system, TALEN, and ZFN. According to the CRISPR/Cas9 system, the genome editing is possible if a guide RNA and a Cas9 protein is present in a target cell. According to TALEN and ZFN, the genome editing is possible if a fusion protein (in which DNA-binding domains and a nuclease are fused) is present in a target cell. Therefore, the guide RNA, the Cas9 protein, and the fusion protein can be directly introduced into a target cell. Examples of a method of directly introducing any of these into the target cell encompass a PEG method, an electroporation method, and a particle bombardment method. Alternatively, a vector in which a construct (including (i) a polynucleotide which encodes the guide RNA and the Cas9 protein and (ii) any promoter and/or any terminator) is inserted may be introduced into the target cell and a tissue via, for example, *Agrobacterium*.

In the case of the CRISPR/Cas9 system, a sequence which is complementary to a nucleotide sequence located immediately upstream of XGG in a genome forms a base pair with part of the guide RNA, and a double-stranded genomic DNA is cleaved by Cas9 in the nucleotide sequence. Examples of the nucleotide sequence encompass 10 or more consecutive bases (e.g., 15 or more consecutive bases, preferably 17 or more consecutive bases, more preferably 18 or more consecutive bases, still more preferably 19 or more consecutive bases, and most preferably 20 or more consecutive bases) located immediately upstream of XGG, in a part of the first polynucleotide or the second polynucleotide.

In the case of TALEN, a pair of DNA-binding domains in artificial nucleases forming a dimer each bind to a corresponding one of nucleotide sequences such that each of the nucleotide sequences is present at a terminus of a corresponding one of FokI cleavage domains so as to be away from the terminus by a spacer of 5 to 20 bases. One of the nucleotide sequences is present at one of strands of double-stranded genomic DNA, and the other of the nucleotide sequences is present at the other of the strands of the double-stranded genomic DNA. Therefore, one of the pair of DNA-binding domains binds to one of the strands, and the other of the pair of DNA-binding domains binds to the other of the strands. Each of the DNA-binding domains is composed of repeating units (modules) each of which is composed of 33 to 34 amino acid residues. The number of modules corresponds to the number of bases to which the DNA-binding domain binds. The nucleotide sequence to which the DNA-binding domain binds is 10 or more consecutive bases, preferably 14 or more consecutive bases, and more preferably 18 or more consecutive bases, which are present at each terminus of a FokI cleavage domain so as to be away from the terminus by a spacer of 5 to 20 bases and which are in a part of a first polynucleotide or a second polynucleotide or a polynucleotide that forms a complementary strand with the first or second polynucleotide.

In the case of ZFN, as in the case of TALEN, a pair of DNA-binding domains in artificial nucleases forming a dimer each bind to a corresponding one of nucleotide sequences such that each of the nucleotide sequences is present at a terminus of a corresponding one of FokI cleavage domains so as to be away from the terminus by a spacer of 5 to 20 bases. Each of the DNA-binding domains is composed of a plurality of zinc finger modules. The nucleotide sequence is 9 or more consecutive bases, preferably 12 or more consecutive bases, and more preferably 18 or more consecutive bases, which are present at each terminus of a FokI cleavage domain so as to be away from the terminus by a spacer of 5 to 20 bases and which are in a part of a first polynucleotide or a second polynucleotide or a polynucleotide that forms a complementary strand with the first or second polynucleotide.

The descriptions of CRISPR/Cas9 system, TALEN, and ZFN, and RNAi (described later) can each be read so that, according to the description of each detail, the polynucleotide having a nucleotide sequence represented by SEQ ID NO: 3 or 4 is replaced with a polynucleotide which has a homologous gene and is present in another kind included in tobacco plants and which has a sequence identity of 95% or higher with the polynucleotide having a nucleotide sequence represented by SEQ ID NO: 3 or 4.

As described above, the mutation, which causes suppression of a function of each of the above endogenous genes and which is introduced in the tobacco plant, is preferably genetically inherited. However, an exogenous polynucleotide introduced in a tobacco plant for genome editing is preferably eliminated from the tobacco plant after it is confirmed that a desired mutation is introduced in the tobacco plant. In a case where the exogenous polynucleotide is retained in the tobacco plant, an undesired mutation may be introduced or may continue to be introduced. This may cause a desired character (such as an increase in content of apocarotenoid) to be lost.

The introduction of the mutation into the endogenous genes of a tobacco plant or the disruption of the endogenous genes of the tobacco plant can be achieved through another biotechnological method (e.g., a method in which a transposon or Agrobacterium is utilized). Concrete examples of the method encompass a method in which (i) a retrotransposon tnt1 of tobacco or a transposon of another plant or (ii) T-DNA of Ti plasmid of Agrobacterium is introduced into a tobacco plant.

Alternatively, the introduction or the disruption can be achieved through another method (mutagen treatment of a tobacco plant). Examples of a source of the mutation encompass small molecule compounds (such as ethyl methane sulfonate (EMS), N-ethyl-N-nitrosourea (ENU), and sodium azide) and radiations (such as gamma rays, heavy ion beams, X-rays, neutron beams, and ultraviolet rays).

A mutation can be introduced into any regenerable tobacco plant. Examples of the tobacco plant encompass seeds, roots, leaves, flowers, reproductive organs, and embryos. A preferable example is seeds.

What can be obtained by the methods above can be a mutant population of a plant which has various mutations (or no mutation). Therefore, an individual exhibiting a desired phenotype can be further selected from the mutant population. As an example of the selection of an individual, the following description will discuss a procedure for selecting a desired individual from a mutant population (panel) which is obtained in a case where tobacco is treated with use of a mutagen.

A tobacco mutant, which has mutations in a total of four alleles in both a T genome and an S genome and the function of which is impaired, can be obtained by, for example, the following method. A tobacco plant is treated with a mutagen as described above to prepare a population (panel) of tobacco mutants with mutations in the whole tobacco genome, and genomic DNAs are extracted. By utilizing gene-specific primers of each of the S genome and the T genome, target genes (polynucleotide) are amplified from the genomic DNAs of the panel. Subsequently, nucleotide sequences of resulting products are determined, and a line having a homozygous mutation is then selected. First, a line (M2) having a homozygous mutation in the S genome and a line (M2) having a homozygous mutation in the T genome are obtained and then crossed to prepare F1 individuals. Subsequently, selfed progeny (F2) is developed from the F1 individuals. From the selfed progeny (F2), a line having homozygous mutations in both the S genome and the T genome is obtained (such a line is obtained at a probability of 1/16 since two factors are recessive).

Another example of carrying out the step of introducing a mutation in a genome of a tobacco plant is suppressed expression of the gene and introduction of the mutation into the gene, which are performed through transformation of the tobacco plant with use of a vector.

The vector used to transform the tobacco plant for the purpose of suppression of expression of the endogenous genes or introduction of the mutation into the endogenous genes is not limited to any particular one, provided that a polynucleotide which is inserted in the vector can be expressed in a plant cell. Suitable examples of the vector encompass pBI, pPZP, and pSMA vectors each of which allows introduction of a target polynucleotide into a plant cell via Agrobacterium. In particular, plasmids of binary vectors (e.g., pBIG, pBIN19, pBI101, pBI121, pBI221, and pPZP202) are preferable.

In a case where suppression of expression of the endogenous genes is achieved by RNAi, a trigger sequence, which is used by RNAi to suppress expression of a target gene, is inserted into the vector. Examples of the trigger sequence encompass (i) a polynucleotide (sense RNA portion) which is (a) a part of a polynucleotide (which can have substitution of 0.1% to 1%) encoding a polypeptide having the amino acid sequence represented by SEQ ID NO: 1 or 2, or a part of a polynucleotide (which can have substitution of 0.1% to 1%) having a nucleotide sequence represented by SEQ ID NO: 3 or 4 and (b) represented by a nucleotide sequence of at least 21 to 30 consecutive bases (e.g., 21 or more bases, 22 or more bases, 23 or more bases, 24 or more bases, 25 or more bases, 26 or more bases, 27 or more bases, 28 or more bases, 29 or more bases, and 30 or more bases) and (ii) a polynucleotide (antisense RNA portion) which is represented by a nucleotide sequence that is complementary to the polynucleotide (i). More specifically, the nucleotide sequence of the "at least 21 to 30 consecutive bases" described above means a nucleotide sequence of 21 or more consecutive bases, 23 or more consecutive bases, 25 or more consecutive bases, 30 or more consecutive bases, 35 or more consecutive bases, 40 or more consecutive bases, 45 or more consecutive bases, 50 or more consecutive bases, 60 or more consecutive bases, 70 or more consecutive bases, 80 or more consecutive bases, 90 or more consecutive bases, or 100 or more consecutive bases.

As described above, the suppression of expression of the endogenous genes in the tobacco plant is preferably genetically inherited. Therefore, the trigger sequence is preferably incorporated in a genome of the tobacco plant.

A tobacco plant, in which expression of a plurality of endogenous genes is simultaneously suppressed, can be obtained by crossing two tobacco plants in which expression of differing endogenous genes is suppressed. In addition, a tobacco plant, in which expression of a plurality of endogenous genes is simultaneously suppressed, can be obtained by (i) performing transformation which may cause expression of a plurality of differing endogenous genes to be simultaneously suppressed and then (ii) selecting the tobacco plant in which expression of a plurality of differing endogenous genes is simultaneously suppressed.

Note that in a case where a tobacco plant in which a plurality of endogenous genes are functionally suppressed is to be obtained by use of crossing, (i) one of tobacco plants to be crossed can be prepared by mutation or disruption of an endogenous gene and (ii) the other one of the tobacco plants to be crossed can be prepared by suppressed expression of an endogenous gene by transformation.

The mutation or disruption of the endogenous genes can be determined by detecting the presence/absence of the mutation in the endogenous genes. A method of detecting the mutation in the endogenous genes only needs to allow the determination of the presence/absence of the mutation. Examples of the method encompass (1) a method in which a DNA sequence that has the mutation and that is commercially available is amplified by PCR or the like, and then a DNA nucleotide sequence is directly decoded with use of a sequencer or the like, (2) a method in which a difference in sequence is detected by a difference in distance of electrophoresis by a single strand conformation polymorphism (SSCP) method, (3) a method in which single nucleotide polymorphism (SNP) is detected by a CycleavePCR method, (4) a method in which the presence/absence of the mutation is detected by cleaving a mismatch site(s) with use of T7 Endonuclease I or the like, (5) a cleaved amplified polymorphic sequence (CAPS) method in which the presence/absence of the mutation can be determined by the presence/absence of cleavage by a restriction enzyme treatment, (6) a derived CAPS (dCAPS) method in which a set of primers including a mismatch intentionally is used so that the presence/absence of the mutation can be determined by the presence/absence of cleavage by restriction enzymes, (7) a method (e.g., a PCR method in which a TaqMan probe is used, MassARRAY analysis) in which the presence/absence of the mutation is determined by detecting, with use of a probe which specifically hybridizes to a mutant sequence, whether or not the probe is hybridized, and (8) a method in which, in a case where the mutation is deletion or insertion, the mutation is detected by a difference in mobility of electrophoresis. Alternatively, the mutation in the endogenous genes can be determined by comparing (i) the size or the expression level of a polypeptide which results from modification of the endogenous genes with (ii) that of a wild-type protein. Specifically, such a comparison can be made by carrying out by, for example, a Western blotting method.

### [3. Cured leaf]

A cured leaf in accordance with an aspect of the present invention is a cured leaf of the tobacco plant. As a method of curing a leaf of the tobacco plant, any method can be employed. The curing method can be, but is not limited to, air curing, warm-air curing, flue curing, or the like, for example. Note that the cured leaf herein encompasses cut fillers, powders, sheets, stems, granules, and extracts each of which is obtained from the cured leaf. The cured leaf can be mixed with other tobacco leaf, material, and the like, and can have a desired taste and in a desired form.

The cured leaf in accordance with an embodiment of the present invention has a higher content of apocarotenoid and an improved aroma and taste (quality), as compared with a cured leaf of a conventional tobacco plant.

### [4. Tobacco product]

A tobacco product in accordance with an aspect of the present invention includes the cured leaf. The tobacco product can be in any form. The tobacco product can be, but is not limited to, shred tobaccos, cigars, pipe smoking tobaccos, paper-wrapped cigarettes, electronic tobaccos, hookah tobaccos, snuff tobaccos (including snus and snuff), heat-not-burn tobacco products (using, as an aerosol source, aerosol generated by heating of tobacco), non-heated tobacco products (for inhaling a flavor of tobacco without heating the tobacco), or the like, for example.

### [5. Tobacco material]

A tobacco material in accordance with an aspect of the present invention is a processed product of a cured leaf or a fresh leaf of the tobacco plant. The tobacco material can be, for example, a product obtained as a result of a grinding process, a fine process, or a shredding process of a cure leaf or a fresh leaf of the tobacco plant. An embodiment of the present invention also encompasses a tobacco product containing the tobacco material.

Aspects of the present invention can also be expressed as follows:
Embodiments of the present invention can be summarized as follows.
(1) A tobacco plant in which a mutation that causes suppression of a function of a first endogenous gene and a function of a second endogenous gene is introduced in a genome, the first endogenous gene containing, as a coding region, a first polynucleotide that encodes a first polypeptide having a sequence identity of 95% or higher with an amino acid sequence represented by SEQ ID NO: 1; and the second endogenous gene containing, as a coding region, a second polynucleotide that encodes a second polypeptide having a sequence identity of 95% or higher with an amino acid sequence represented by SEQ ID NO: 2.
(2) The tobacco plant as set forth in (1), wherein the suppression of the function is a decrease in an amount of mRNA which has been transcribed from the first endogenous gene and the second endogenous gene, as compared with a wild-type plant.
(3) The tobacco plant as set forth in (2), wherein the suppression of the function is promotion of degradation of mRNA which has been transcribed from the first endogenous gene and the second endogenous gene.
(4) The tobacco plant as set forth in any one of (1) to (3), wherein the suppression of the function is a decrease in an amount of the first polypeptide and the second polypeptide which are original functional polypeptides, as compared with a wild-type plant.
(5) The tobacco plant as set forth in (4), wherein the suppression of the function is a decrease in an amount of translation into the first polypeptide and the second polypeptide which are original functional polypeptides, as compared with a wild-type plant.
(6) The tobacco plant as set forth in any one of (1) to (5), wherein in at least one endogenous gene selected from the group consisting of the first endogenous gene and the second endogenous gene, alleles have a same type of mutation.
(7) The tobacco plant as set forth in any one of (1) to (6), wherein the mutation is introduced by mutagen treatment, genome editing, or gene knockout.
(8) The tobacco plant as set forth in any one of (1) to (7), wherein the tobacco plant belongs to Nicotiana tabacum or Nicotiana rustica.
(9) A method for producing a tobacco plant product, the method comprising a step of introducing, in a genome of the tobacco plant, a mutation that causes suppression of a function of a first endogenous gene and a function of a second endogenous gene, the first endogenous gene containing, as a coding region, a first polynucleotide that encodes a first polypeptide having a sequence identity of 95% or higher with an amino acid sequence represented by SEQ ID NO: 1; and the second endogenous gene containing, as a coding region, a second polynucleotide that encodes a second polypeptide having a sequence identity of 95% or higher with an amino acid sequence represented by SEQ ID NO: 2.
(10) The method as set forth in (9), wherein the suppression of the function is a decrease in an amount of mRNA which has been transcribed from the first endogenous gene and the second endogenous gene, as compared with a wild-type plant.
(11) The method as set forth in (10), wherein the suppression of the function is promotion of degradation of mRNA which has been transcribed from the first endogenous gene and the second endogenous gene.
(12) The method as set forth in any one of (9) to (11), wherein the suppression of the function is a decrease in an amount of the first polypeptide and the second polypeptide which are original functional polypeptides, as compared with a wild-type plant.
(13) The method as set forth in (12), wherein the suppression of the function is a decrease in an amount of translation into the first polypeptide and the second polypeptide which are original functional polypeptides, as compared with a wild-type plant.
(14) The method as set forth in any one of (9) to (13), wherein in the tobacco plant, alleles in at least one endogenous gene selected from the group consisting of the first endogenous gene and the second endogenous gene have a same type of mutation.
(15) The method as set forth in any one of (9) to (14), wherein the step of introducing the mutation is carried out by mutagen treatment, genome editing, or gene knockout.
(16) An offspring or bred progeny, the offspring being of a tobacco plant recited in any one of (1) to (8) or a tobacco plant produced by a method recited in any one of (9) to (15), the bred progeny being obtained by crossing a tobacco plant recited in any one of (1) to (8) or a tobacco plant produced by a method recited in any one of (9) to (15) with another tobacco plant.
(17) A cured leaf having: a mutation in a first endogenous gene in a genome, the first endogenous gene containing, as a coding region, a first polynucleotide that encodes a first polypeptide having a sequence identity of 95% or higher with an amino acid sequence represented by SEQ ID NO: 1, the mutation suppressing a function of the first endogenous gene; and a mutation in a second endogenous gene in a genome, the second endogenous gene containing, as a coding region, a second polynucleotide that encodes a second polypeptide having a sequence identity of 95% or higher with an amino acid sequence represented by SEQ ID NO: 2, the mutation suppressing a function of the second endogenous gene.
(18) A tobacco product comprising a cured leaf recited in (17).

### Examples

### [1. Preparation of tobacco plant having mutation in LCY-e gene]

Lines having a mutation in an LCY-e-S gene or an LCY-e-T gene were selected from an EMS mutant population (M2 generation, approximately 2000 lines) of a tobacco cultivar (Tsukuba 1) according to sequence determination by amplicon sequencing. As lines having a mutation in the LCY-e-S gene, 2 lines (LCY-e-S-1: Line 1461 and LCY-e-S-2: Line 69) were obtained. In each of these 2 lines, a single homogenous nonsense mutation occurred. In Line 1461, a codon encoding the 78th glutamine (Q) of an LCY-e-S protein has been altered to a termination codon by a nucleotide substitution. In Line 69, a codon encoding the 145th tryptophan (W) of an LCY-e-S protein has been altered to a termination codon by a nucleotide substitution. As a line having a mutation in an LCY-e-T gene, Line 1944 in which a single homogenous nonsense mutation occurred in the gene was obtained. In Line 1944, a codon encoding the seventh arginine (R) of an LCY-e-T protein has been altered to a termination codon by a nucleotide substitution. Hereinafter, Line 1944 may also be referred to as LCY-e-T-1.

The above-described 3 lines were used to prepare tobacco double mutants having mutations in both the LCY-e-S gene and the LCY-e-T gene. An F1 plant obtained by crossing between Line 1461 and Line 1944 through artificial pollination was selfed, so that an F2 generation population was obtained. From the F2 generation population, an LCY-e-1 line having homogenous mutations in both of two LCY-e genes was selected according to sequence determination by amplicon sequencing. An F1 plant obtained by crossing between Line 69 and Line 1944 through artificial pollination was selfed, so that an F2 generation population was obtained. From the F2 generation population, an LCY-e-2 line having homogenous mutations in both of two LCY-e genes was selected according to sequence determination by amplicon sequencing.

The following primers were used for the amplicon sequencing.
LCY-e-S-1 forward primer: AAGTGATAGTTGTGTAGTG (SEQ ID NO: 7)
LCY-e-S-1 reverse primer: CTGATCCTGACATAACTTG (SEQ ID NO: 8)
LCY-e-S-2 forward primer: ACTGGATTTAGTGGTCATT (SEQ ID NO: 9)
LCY-e-S-2 reverse primer: AAGCTCCAGAGAACAAGAG (SEQ ID NO: 10)
LCY-e-T-1 forward primer: AAAACAGAGAAGTCAGAT (SEQ ID NO: 11)
LCY-e-T-1 reverse primer: CTTCACTTGCATATGTAT (SEQ ID NO: 12)

### [2. Quantitative analysis of apocarotenoids]

Seedlings for transplantation of the double mutants (LCY-e-1 and LCY-e-2) of the 2 lines, which had been obtained in [1. Preparation of tobacco plant having mutation in LCY-e gene], and an original variety (Tsukuba 1) were grown in a greenhouse. After transplantation into a field, cultivation was carried out under common cultivation conditions for leaf tobaccos. Five leaves were harvested from each plant, 48 days to 55 days after topping. Then, the leaves were subjected to a common flue curing treatment with use of a flue curer. From cured leaves thus obtained, mid-rib portions were removed. Then, lamina portions were subjected to a grinding treatment, so that a ground sample was prepared. Into a 14-mL screw-mouth glass centrifuge tube, 0.1 g of the ground sample and 6.0 mL of an extraction solution (methanol to which 1,3-dimethoxybenzene (0.53 µg/mL) that was used as an internal standard substance had been added) were introduced. Then, the centrifuge tube was covered with a screw cap. Thereafter, shake extraction (at 250 rpm at 70°C for 1 hour) was carried out with use of a medium-sized constant temperature incubator shaker Bioshaker (registered trademark) BR-43FH·MR (TAITEC Corp.). A resultant extract solution was filtered through a PTFE filter having a pore size of 0.45 µm and subjected to analysis by a gas chromatography-mass spectrometer (GC/MS). The following are devices, instruments and conditions used for the analysis.
- Gas chromatography-mass spectrometer: GC/MS 5977A MSD (Agilent Technologies, Inc., Tokyo)
- Column: HP-1 ms (internal diameter: 0.25 mm, length: 30 m, membrane thickness: 0.25 µm; Agilent Technologies, Inc., Tokyo)
- Insert liner: splitless liner with inert glass wool (Agilent Technologies, Inc., Tokyo)
- Helium flow rate: 1.0 mL/min
- Injection volume: 1.0 µL
- Injection mode: Splitless
- Insert temperature: 280°C
- AUX temperature: 280°C
- Programmed column temperature conditions:
   (1) Retain at 50°C for 1 minute
   (2) Increase temperature from 50°C to 100°C at 10°C/min
   (3) Increase temperature from 100°C to 200°C at 2°C/min
   (4) Increase temperature from 200°C to 300°C at 20°C/min
   (5) Retain at 300°C for 19 minutes
- Analysis mode: TIC/SIM mode.

Analysis of a TIC was carried out in a range of 30 m/z to 500 m/z. A combination of a semiquantitative ion and a qualitative ion (SIM parameters) of each apocarotenoid to be analyzed in the SIM was set as shown in Table 1. In Table 1, the number in parentheses appended to the end of a compound indicates that the compound is a structural isomer.

**[Table 1]**

| Compound name | Retention time (min) | Semiquantitative ion | Qualitative ion | |
|---|---|---|---|---|
| 1,3-dimethoxybenzene (internal standard) | 9.8 | 138 | 109 | 95 |
| β-cyclocitral | 11.6 | 137 | 152 | 123 |
| β-damascenone | 18.1 | 69 | 121 | 190 |
| β-damascone | 19.5 | 177 | 192 | 123 |
| Oxo-edulan (2) | 21.2 | 193 | 109 | 208 |
| Oxo-edulan (1) | 22.4 | 193 | 109 | 208 |
| 8,9-dehydrotheaspirone | 22.7 | 108 | 206 | 150 |
| β-ionone | 22.9 | 177 | 192 | |
| Dihydroactinidiolide | 23.6 | 111 | 137 | 180 |
| Megastigmatrienone (1) | 26.3 | 190 | 175 | 147 |
| Megastigmatrienone (2) | 27.3 | 190 | 175 | 147 |
| 3-hydroxy-β-damascone | 28.8 | 175 | 193 | 208 |
| Megastigmatrienone (4) | 28.8 | 190 | 175 | 147 |
| Megastigmatrienone (3) | 29.5 | 190 | 175 | 147 |
| 3-hydroxy-7,8-dehydro-β-ionol | 29.9 | 193 | 208 | 175 |
| 3-oxo-α-ionol | 30.3 | 108 | 152 | |
| 3-hydroxy-5,6-epoxy-β-ionol | 31.6 | 125 | 208 | 43 |
| Blumenol_C | 33.0 | 135 | 150 | 210 |
| 4-(3-hydroxybuthylidene)-3,5,5-trimethyl-2-cyclohexen-1-one | 33.5 | 149 | 164 | |
| Blumenol_A | 37.1 | 124 | 135 | 168 |

Each apocarotenoid component was identified by matching, with literature information and information in a database, a full-mass spectrum that was obtained in the TIC mode in a retention time in which the semiquantitative ion and the qualitative ion were detected in the SIM mode. The content of each apocarotenoid was calculated. This calculation employed, as a semiquantitative value, a relative value of an area value in a chromatogram of the semiquantitative ion of the apocarotenoid to an area value in a chromatogram of the semiquantitative ion of 1,3-dimethoxybenzene which was an internal standard substance. Respective quantities of apocarotenoids in each line were summarized in Table 2. Table 2 shows an apocarotenoid content for one line as an average value of values indicating contents of the apocarotenoid in three samples obtained from 3 plots of the field. Table 2 only shows quantities of only apocarotenoids that have been detected in the samples. As shown in Table 2, contained were (a) apocarotenoids which were increased by 1.2 times to 3.1 times commonly in LCY-e-1 and LCY-e-2 (lines 1-9 in Table 2) as compared with the control Tsukuba 1 (Tsukuba 1) and (b) apocarotenoids did not increase commonly (lines 10-16 in Table 2) as compared with a control Tsukuba 1 (Tsukuba 1). Nine species that showed the above increase included β-damascenone (1.2 times to 1.4 times), β-damascone (1.4 times to 1.6 times), and β-ionone (1.5 times to 1.7 times), each of which is known as an aromatic apocarotenoid.

**[Table 2]**

| | LCY-e-1 | LCY-e-2 | Tsukuba1 |
|---|---|---|---|
| Dihydroactinidiolide | 0.0574 | 0.0532 | 0.0257 |
| β-ionone | 0.0391 | 0.0356 | 0.0232 |
| β-damascenone | 0.0733 | 0.0619 | 0.0525 |
| β-damascone | 0.0387 | 0.0350 | 0.0242 |
| 3-hydroxy-β-damascone | 0.5288 | 0.5268 | 0.4517 |
| 3-hydroxy-5,6-epoxy-β-ionol | 0.2018 | 0.1197 | 0.0654 |
| β-cyclocitral | 0.0112 | 0.0125 | 0.0085 |
| 3-hydroxy-7.8-dehydro-β-ionol | 0.0229 | 0.0152 | 0.0118 |
| Blumenol_A | 0.2996 | 0.1794 | 0.1408 |
| Blumenol_C | 0.0164 | 0.0117 | 0.0150 |
| 3-oxo-α-ionol | 0.4356 | 0.3444 | 0,4765 |
| 4-(3-hydroxybuthylidene)-3,5,5-trimethyl | 0.0413 | 0.0255 | 0.0223 |
| Megastigmatrienone(1) | 0.2847 | 0.2853 | 0.2671 |
| Megastigmatrienone(2) | 0.8742 | 0.8864 | 0.8385 |
| Megastigmatrienone(3) | 0.7631 | 0.7902 | 0.7557 |
| Megastigmatrienone(4) | 0.2649 | 0.2678 | 0.2537 |

### [3. Sensory evaluation test on finely ground sample]

Cured leaves of leaves that had been harvested from the double mutants (LCY-e-1 and LCY-e-2) of 2 lines grown in the field and the tobacco cultivar (Tsukuba 1) were ground and further a fine grinding treatment. The leaves having been subjected to the fine grinding treatment were dispersed into propylene glycol (having a mass that was twice that of the leaved having subjected to the fine grinding treatment), so that finely ground samples were prepared. A sensory evaluation (comparison of a test object with a control object) was carried out by five trained panelists. The control object was a non-aromatized product of MEVIUS (registered trademark). The test object was a product obtained by spreading each finely ground sample on a non-aromatized product of MEVIUS (registered trademark). The panelists were requested to smoke the control object and the test object, and to answer how aroma and taste of the test object differ from those of the control object. Table 3 summarizes results of the following scoring for each test objects: one (1) point was given in a case where a panelist answered that an improvement in floral aroma and taste was perceived; and zero (0) points were given in a case where a panelist answered that an improvement in floral aroma and taste was perceived but weak, or no improvement was perceived. As shown in Table 3, in the case of the LCY-e-1, 4 out of 5 panelists perceived an in floral aroma and taste, and in the case of the LCY-e-2, 3 out of 5 panelists perceived improvement of floral aroma and taste. The above results indicate that cured leaves of the leaves that had been harvested from the LCY-e double mutant can add favorable aroma and taste to a conventional tobacco product.

**[Table 3]**

| | LCY-e-1 | LCY-e-2 | Tsukuba1 |
|---|---|---|---|
| Total score | 4 | 3 | 0 |

### [4. Comparison between double mutant and single mutant]

Seedlings for transplantation of the double mutants (LCY-e-1 and LCY-e-2) of the 2 lines and single mutants (LCY-e-S-1, LCY-e-S-2, LCY-e-T-1 and LCY-e-T-2), which had been obtained in [1. Preparation of tobacco plant having mutation in LCY-e gene], and the original variety (Tsukuba 1) were grown in a greenhouse. LCY-e-T-2 is a line (Line 367) in which a single nonsense mutation occurred homologously in an LCY-e-T gene. In the LCY-e-T-2, a codon encoding the 78th glutamine (Q) of an LCY-e-T protein has been altered to a termination codon by a nucleotide substitution.

In selection of the LCY-e-T-2, the following primers were used for amplicon sequencing.
LCY-e-T-2 forward primer: AAGTGTAGTGGAAATGAGA (SEQ ID NO: 13)
LCY-e-T-2 reverse primer: AATACTGCTTCCGTCACTG (SEQ ID NO: 14)

Except that five leaves that had been harvested from each plant, 39 days to 52 days after topping, were subjected to a conventional flue curing treatment using a flue curer, quantitative determination was carried out, for respective quantities of apocarotenoids contained in each line, under the conditions that were the same as those in [2. Quantitative analysis of apocarotenoids]. Fig. 1 and Table 4 show this quantitative determination results.

The vertical axis of Fig. 1 represents the quantity of apocarotenoid in each line, in a case where the quantity of apocarotenoid in Tsukuba 1 is set to 1. Table 4 shows the respective quantities of apocarotenoids contained in each line.

**[Table 4]**

| | Double mutant | Single mutant | | | | Cultivar |
|---|---|---|---|---|---|---|
| | LCY-e-2 | LCY-e-S-1 | LCY-e-S-2 | LCY-e-T-1 | LCY-e-T-2 | Tsukuba1 |
| Dihydroactinidiolide | 0.0863 | 0.1602 | 0.1121 | 0.0988 | 0.0648 | 0.0552 |
| β-ionone | 0.0621 | 0.0569 | 0.0606 | 0.0514 | 0.0521 | 0.0508 |
| β-damascenone | 0.2082 | 0.1191 | 0.1228 | 0.1511 | 0.1395 | 0.1457 |
| β-damascone | 0.0452 | 0.0372 | 0.0361 | 0.0319 | 0.0328 | 0.0325 |
| 3-hydroxy-β-damascone | 1.0680 | 0.9918 | 1.0001 | 1.0191 | 1.0980 | 0.9794 |
| 3-hydroxy-5,6-epoxy-β-ionol | 0.2363 | 0.2546 | 0.2209 | 0.1329 | 0.2511 | 0.1053 |
| β-cyclocitral | 0.0341 | 0.0211 | 0.0232 | 0.0211 | 0.0211 | 0.0193 |
| 3-hydroxy-7,8-dehydro-β-ionol | 0.0214 | 0.0261 | 0.0212 | 0.0160 | 0.0192 | 0.0155 |
| Blumenol_A | 0.2869 | 0.5528 | 0.3225 | 0.2179 | 0.2914 | 0.1730 |
| Blumenol_C | 0.0300 | 0.0571 | 0.0360 | 0.0380 | 0.0365 | 0.0313 |
| 3-oxo-α-ionol | 0.3571 | 1.3413 | 0.7580 | 0.5009 | 0.6497 | 0.4085 |
| 4-(3-hydroxybuthylidene)-3,5,5-trimethyl-2-cyclohexen-1-one | 0.0627 | 0.0714 | 0.0685 | 0.0385 | 0.0599 | 0.0294 |
| Megastigmatrienone(1) | 0.4871 | 0.5088 | 0.4948 | 0.5509 | 0.5463 | 0.5130 |
| Megastigmatrienone(2) | 1.3712 | 1.4997 | 1.4332 | 1.6243 | 1.5595 5 | 1.4800 |
| Megastigmatrienone(3) | 2.0193 | 2.1955 | 2.1298 | 2.3921 | 2.2991 | 2.1732 |
| Megastigmatrienone(4) | 0.6630 | 0.7090 | 0.6802 | 0.7445 | 0.7380 | 0.6781 |

As shown in Table 4, there were components (e.g., dihydroactinidiolide) that were increased commonly in the single mutants (1.2 times to 2.9 times) and the double mutant (1.6 times) as compared with the cultivar Tsukuba 1. On the other hand, as illustrated in Fig. 1 and Table 4, there were also components (e.g., β-damascenone, β-damascone, and β-cyclocitral) that were increased only in the double mutant. In the case of β-damascenone, in a comparison with the cultivar Tsukuba 1, the double mutant increased by 1.4 times whereas the single mutant increased by 0.8 times to 1.0 time. Thus, an increase was found only in the double mutant. Similarly, in the case of β-damascone, in a comparison with the cultivar Tsukuba 1, the double mutant increased by 1.4 times whereas the single mutant increased by 1.0 time to 1.1 times. Thus, an increase was found only in the double mutant. Similarly, in the case of β-cyclocitral, in a comparison with the cultivar Tsukuba 1, the double mutant increased by 1.8 times whereas the single mutant increased by 1.1 time to 1.2 times. Thus, an increase was observed only in the double mutant. β-cyclocitral is known as one aroma apocarotenoid component.

### Industrial Applicability

An embodiment of the present invention can be used for improving quality of tobacco products.

## Claims

1. A tobacco plant in which a mutation that causes suppression of a function of a first endogenous gene and a function of a second endogenous gene is introduced in a genome,
the first endogenous gene containing, as a coding region, a first polynucleotide that encodes a first polypeptide having a sequence identity of 95% or higher with an amino acid sequence represented by SEQ ID NO: 1; and
the second endogenous gene containing, as a coding region, a second polynucleotide that encodes a second polypeptide having a sequence identity of 95% or higher with an amino acid sequence represented by SEQ ID NO: 2.

2. The tobacco plant as set forth in claim 1, wherein the suppression of the function is a decrease in an amount of mRNA which has been transcribed from the first endogenous gene and the second endogenous gene, as compared with a wild-type plant.

3. The tobacco plant as set forth in claim 2, wherein the suppression of the function is promotion of degradation of mRNA which has been transcribed from the first endogenous gene and the second endogenous gene.

4. The tobacco plant as set forth in any one of claims 1 to 3, wherein the suppression of the function is a decrease in an amount of the first polypeptide and the second polypeptide which are original functional polypeptides, as compared with a wild-type plant.

5. The tobacco plant as set forth in claim 4, wherein the suppression of the function is a decrease in an amount of translation into the first polypeptide and the second polypeptide which are original functional polypeptides, as compared with a wild-type plant.

6. The tobacco plant as set forth in any one of claims 1 to 5, wherein in at least one endogenous gene selected from the group consisting of the first endogenous gene and the second endogenous gene, alleles have a same type of mutation.

7. The tobacco plant as set forth in any one of claims 1 to 6, wherein the mutation is introduced by mutagen treatment, genome editing, or gene knockout.

8. The tobacco plant as set forth in any one of claims 1 to 7, wherein the tobacco plant belongs to *Nicotiana tabacum* or *Nicotiana rustica.*

9. A method for producing a tobacco plant product, the method comprising a step of introducing, in a genome of the tobacco plant, a mutation that causes suppression of a function of a first endogenous gene and a function of a second endogenous gene,
the first endogenous gene containing, as a coding region, a first polynucleotide that encodes a first polypeptide having a sequence identity of 95% or higher with an amino acid sequence represented by SEQ ID NO: 1; and
the second endogenous gene containing, as a coding region, a second polynucleotide that encodes a second polypeptide having a sequence identity of 95% or higher with an amino acid sequence represented by SEQ ID NO: 2.

10. The method as set forth in claim 9, wherein the suppression of the function is a decrease in an amount of mRNA which has been transcribed from the first endogenous gene and the second endogenous gene, as compared with a wild-type plant.

11. The method as set forth in claim 10, wherein the suppression of the function is promotion of degradation of mRNA which has been transcribed from the first endogenous gene and the second endogenous gene.

12. The method as set forth in any one of claims 9 to 11, wherein the suppression of the function is a decrease in an amount of the first polypeptide and the second polypeptide which are original functional polypeptides, as compared with a wild-type plant.

13. The method as set forth in claim 12, wherein the suppression of the function is a decrease in an amount of translation into the first polypeptide and the second polypeptide which are original functional polypeptides, as compared with a wild-type plant.

14. The method as set forth in any one of claims 9 to 13, wherein in the tobacco plant, alleles in at least one endogenous gene selected from the group consisting of the first endogenous gene and the second endogenous gene have a same type of mutation.

15. The method as set forth in any one of claims 9 to 14, wherein the step of introducing the mutation is carried out by mutagen treatment, genome editing, or gene knockout.

16. An offspring or bred progeny, the offspring being of a tobacco plant recited in any one of claims 1 to 8 or a tobacco plant produced by a method recited in any one of claims 9 to 15, the bred progeny being obtained by crossing a tobacco plant recited in any one of claims 1 to 8 or a tobacco plant produced by a method recited in any one of claims 9 to 15 with another tobacco plant.

17. A cured leaf having:
a mutation in a first endogenous gene in a genome, the first endogenous gene containing, as a coding region, a first polynucleotide that encodes a first polypeptide having a sequence identity of 95% or higher with an amino acid sequence represented by SEQ ID NO: 1, the mutation suppressing a function of the first endogenous gene; and
a mutation in a second endogenous gene in a genome, the second endogenous gene containing, as a coding region, a second polynucleotide that encodes a second polypeptide having a sequence identity of 95% or higher with an amino acid sequence represented by SEQ ID NO: 2, the mutation suppressing a function of the second endogenous gene.

18. A tobacco product comprising a cured leaf recited in claim 17.
